# EUROPEAN PATENT SPECIFICATION

(11) **EP 4 238 412 B1**
(45) Date of publication and mention of the grant of the patent: **27.08.2025**
(21) Application number: 20824959.9
(22) Date of filing: 30.10.2020
(51) Int. Cl.: A01K 29/00, A01K 67/30, G06M 11/00, G06M 7/00, G06M 1/02

(54) **AUTOMATED HIGH-RESOLUTION PUPAE COUNTING DEVICE**
AUTOMATISCHE HOCHAUFLÖSENDE PUPAE-ZÄHLVORRICHTUNG
DISPOSITIF DE DÉNOMBREMENT DE PUPES À HAUTE RÉSOLUTION AUTOMATISÉ

(43) Date of publication of application: 06.09.2023
(73) Proprietor: Consejo Superior De Investigaciones Científicas, 28006 Madrid (ES); Universidad Miguel Hernandez, 03202 Elche, Alicante (ES)
(72) Inventor: SANTORO, Roberto, 03550 San Juan de Alicante (Alicante) (ES); DOMINGUEZ CASTELLANO, María, 03550 San Juan de Alicante (Alicante) (ES); RODRÍGUEZ MILÁN, Víctor Javier, 03550 San Juan de Alicante (Alicante) (ES)
(74) Representative: Pons IP
(86) International application number: PCT/ES2020/070670
(87) International publication number: WO 2022/090589

(56) References cited:
- WO-A2-2004/006854
- WO-A2-2019/008591
- CN-U- 206 033 758

## Description

### OBJECT OF THE INVENTION

The object of the invention is a pupae counting device that allows the automatic quantification of the youth-adult transition in groups of insects, preferably Drosophila flies. It can quantify, in real time and in a precise and reliable manner, the time of development of larva-adult in studies that require monitoring of the real time of initiation of sexual maturity.

### BACKGROUND OF THE INVENTION

Sexual growth and maturation occur through a series of stages of development precisely timed, but also showing plasticity and adaptation in response to certain environmental conditions and endogenous factors, such as disease, poor nutrition, mutations or endocrine disruptors.

There is an enormous interest on clinical factors causing early initiation in the transition between the juvenile to adult stage. The delay or acceleration of this transition can have a negative and persistent impact affecting both the final size and fertility, and is a risk factor for obesity, and certain cancers.

The beginning of sexual maturation (called metamorphosis in insects, and its equivalent, puberty, in mammals) is the most critical transition in the life story of an organism, which maximizes its reproductive success and adaptation to the environment.

The key signal of the beginning of sexual maturation has not been identified in any animal species and as a consequence, the control of this process has been the subject of intense research for decades.

The onset of sexual maturation varies from individual to individual within a species. The age of onset of maturation or metamorphosis/puberty is influenced by environmental factors, such as changes in temperature, and/or length of day (seasons), nutrition, or genetic factors, endocrine disruption or disease.

The youthful organisms do not enter into sexual maturation until they are competent, and have acquired an adequate development and growth. Toxic substances in the environment such as plastic, pollution, infections, malnutrition, infections, and diseases such as tumours and unknown genetic factors can alter the age of puberty, and metamorphosis in insects.

The vinegar fly Drosophila melanogaster is one of the most used insects in research on growth, development, neurogenetics, and physiology of 'puberty'. The rate of development is also an important parameter in studies of efficacy and/or toxicity of drugs and recently this fly has been used for high efficiency drug screening studies in pre-clinical models of neurodegenerative and cancer diseases.

Document WO2004/006854 describes a method for screening for the effect of a test on a population of biological specimens, preferably insects, comprising the steps of providing a population of specimens, administering at least one test agent to the population, creating a digitized movie showing the movements of members of the population and measuring at least one trait of members of the population with the effect of the test agent.

Document CN206033758U describes a rotation platform based automated-high resolution tracking device for monitoring the presence or absence of bacterial growth.

### DESCRIPTION OF THE INVENTION

The invention is defined by the appended claims.

The pupae counting device of the present invention allows the automatic quantification of the youth-adult transition in groups of insects, preferably Drosophila flies. It can quantify, in real time and in a precise and reliable manner, the time of development of larva-adult in studies that require monitoring of the real time of initiation of sexual maturity.

To this end, the device comprises a camera that records video and image, directed towards a platform. On the platform, one or more tube holders, preferably arranged in a circular pattern, hold one or more sample tubes. A central motor is linked to the platform, rotating it in front of the camera, until a sample tube of interest is positioned in front of the camera. An external motor is positioned next to the platform, rotating a sample tube of interest, which is the one closest to the camera. In this way, the sample tube of interest can rotate 360° over itself, allowing the camera to record its entire surface.

The device can also comprise bearings, positioned between the tube holders and the platform, which is preferably circular, allowing their smooth rotation.

In an aspect of the invention, the camera is connected to an external device that runs a software which, using the video and images recorded by the camera, is capable of automatically quantifying the time and number of animals contained in the sample tubes, in different stages of development of the pupae in the youth-adult transition.

The external device can also monitor parameters that can affect experimental results such as food quality, bacterial contamination, etc.

In another aspect of the invention, the device comprises a lighting module, with an infrared and/or white light, that illuminates the sample tubes, in order to recreate day and night.

Preferably, both the motors (central motor and external motor) and the lighting module comprise a housing that covers them with an internal insulator, in order to protect them from humidity and allowing a longer life span of the device.

If the experiment conditions need to be fully controlled, the device can be positioned inside of an incubator, with humidity and temperature sensor and controllers, or any other variable sensor and actuator that could be needed. In this way, all variables of interest for the pupae development can be monitored and controlled when needed.

In this sense, in studies that require monitoring the time of initiation of sexual maturity, the automated high-resolution pupae counting device, object of the invention, saves time and improves accuracy and reproducibility in comparison with manual and visual counting.

Moreover, the device has the following advantages:
- it is robust and durable and can be used in an incubator,
- it is protected against moisture,
- its movements are smooth and its rotations do not interfere with trials or tests of insect behavior,
- it can be adapted to other insects besides Drosophila melanogaster,
- the lighting module allows for circadian cycle experiments by registering and counting flies for twenty-four hours (light and dark) or longer periods (days, weeks), and
- it allows to carry out development time experiments in highly controlled environmental conditions (e.g., humidity level, light, temperature, etc.).

The device can be adapted for high-efficiency experiments, such as the detection of genes, inhibitors, drugs or medicines, which need to count a lot of animals, accurately measure the time of death of the animal, or time its development from the juvenile to the adult stage with great precision.

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figure 1.- Shows a general view of the automated high-resolution pupae counting device.
Figure 2.- Shows an upper view of the device.

### PREFERRED EMBODIMENT OF THE INVENTION

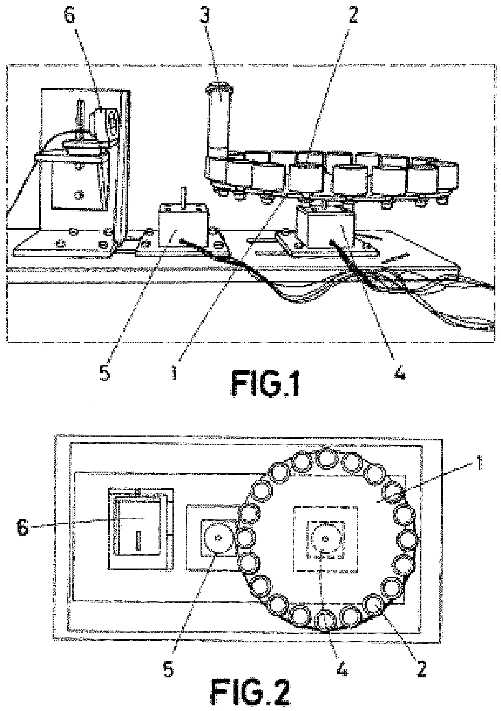

With the help of figures 1 and 2, a preferred embodiment of the automated high-resolution pupae counting device, object of the present invention, is described below.

The device, shown in a general view in figure 1, is intended for automated monitoring and counting of Drosophila development. It allows an automated high-resolution monitoring and counting of larvae and pupae over days to weeks. The device can automatically count hundreds of flies during weeks in a single experiment.

The device comprises a camera (6) for recording video and images, directed towards a circular platform (1), as shown in figure 1. On the platform (1), one or more tube holders (2) are arranged in a circular pattern, as shown in figure 2. A sample tube (3), where the pupas are placed, is inserted into each tube holder (2).

A central motor (4) is linked to the platform (1), rotating it in front of the camera (6), until a sample tube (3) of interest is positioned in front of the camera (6). An external motor (5) is positioned besides the platform (1), rotating the sample tube (3) of interest, which is the one closest to the camera (6). In this way, the sample tube (3) of interest can rotate 360° over itself, allowing the camera (6) to record its entire surface, and therefore all the pupae contained inside.

The central motor (4) and the external motor (5) are stepper motors, facilitating the angular rotation control of both the platform (1) and the sample tubes (3).

The device also comprises bearings, not shown in figure 1, positioned between the sample tubes (3) and the platform (1), and that allow their smooth rotation. The slow movements and the low noise are ideal for the recording of multiple adult-fly genotype. The flies do not notice a thing.

The camera (6) is connected to an external device that, using the video and images recorded by the camera (6), runs a software capable of automatically quantifying the time and number of pupae in different stages of development in the youth-adult transition contained in each sample tube (3). The external device also monitors parameters that can affect experimental results such as food quality, bacterial contamination, etc. For example, food quality can be determined by its color.

The device additionally comprises a lighting module, with an infrared and a white light, that illuminates the sample tubes (3), in order to recreate day and night. The lighting module allows to perform circadian clock experiments.

Both the motors (4, 5) and the lighting module comprise a housing that covers them with an internal insulator, in order to protect them from humidity and allowing a longer life span of the device.

To allow a better adaptability to any kind of experiment, the sample tubes (3) can be any standard fly food vial/tube, not requiring special lab equipment.

The embodiment shown in figure 2 allows the recording of up to twenty sample tubes (3), with the possibility to increase this number for high-efficiency experiments.

## Claims

1. An automated high-resolution pupae counting device that comprises:
- a platform (1),
- one or more tube holders (2) arranged on the platform (1),
- one or more sample tubes (3), inserted into each tube holder (2), and intended to contain pupae,
- a camera (6) for recording video and image, directed towards the platform (1), connected to an external device that quantifies the time and number of pupae contained in each sample tube (3),
- a central motor (4) linked to the platform,
**characterized in that** the central motor (4) rotates the platform (1) until a sample tube (3) of interest is positioned in front of the camera (6), and **in that** the device additionally comprises:
- an external motor (5) positioned beside the platform (1), that rotates the sample tube (3) of interest 360° over itself in front of the camera (6).

2. The device according to claim 1, wherein the platform (1) is circular and the tube holders (2) are arranged in a circle.

3. The device according to claim 1, wherein the central motor (4) and/or the external motor (5) are stepper motors.

4. The device according to claim 1, wherein it additionally comprises bearings, positioned between the tube holders (2) and the platform (1).

5. The device according to claim 1, wherein it additionally comprises a lighting module, that illuminates the sample tubes (3).

6. The device according to claim 5, wherein the lighting module comprises an infrared light and a white light.

7. The device according to claim 5 or 6, wherein the lightings module comprises a housing for humidity protection.

8. The device according to claim 1, wherein the external motor (5) and the central motor (4) comprise a housing for humidity protection.

## Patentansprüche

1. Automatische, hochauflösende Puppenzählvorrichtung, die Folgendes umfasst:
- eine Plattform (1),
- einen oder mehrere auf der Plattform (1) angeordnete Röhrchenhalter (2),
- ein oder mehrere in jeden Röhrchenhalter (2) eingesetzte Probenröhrchen (3) zur Aufnahme von Puppen,
- eine auf die Plattform (1) gerichtete Kamera (6) zur Video- und Bildaufzeichnung, die mit einer externen Vorrichtung verbunden ist, welche die Zeit und Anzahl der in jedem Probenröhrchen (3) enthaltenen Puppen misst,
- einen zentralen Motor (4), der mit der Plattform verbunden ist,
**dadurch gekennzeichnet, dass** der zentrale Motor (4) die Plattform (1) dreht, bis sich ein gewünschtes Probenröhrchen (3) vor der Kamera (6) befindet, und dadurch dass die Vorrichtung zusätzlich Folgendes umfasst:
- einen externen Motor (5), der neben der Plattform (1) positioniert ist und das gewünschte Probenröhrchen (3) vor der Kamera (6) 360° um sich selbst dreht.

2. Vorrichtung nach Anspruch 1, wobei die Plattform (1) kreisförmig ist und die Röhrchenhalter (2) kreisförmig angeordnet sind.

3. Vorrichtung nach Anspruch 1, wobei der zentrale Motor (4) und/oder der externe Motor (5) Schrittmotoren sind.

4. Vorrichtung nach Anspruch 1, wobei sie zusätzlich Lager aufweist, die zwischen den Röhrchenhaltern (2) und der Plattform (1) angeordnet sind.

5. Vorrichtung nach Anspruch 1, **dadurch gekennzeichnet, dass** sie zusätzlich ein Beleuchtungsmodul umfasst, das die Probenröhrchen (3) beleuchtet.

6. Vorrichtung nach Anspruch 5, wobei das Beleuchtungsmodul ein Infrarotlicht und ein Weißlicht umfasst.

7. Vorrichtung nach Anspruch 5 oder 6, wobei das Beleuchtungsmodul ein Gehäuse zum Schutz vor Feuchtigkeit umfasst.

8. Vorrichtung nach Anspruch 1, wobei der externe Motor (5) und der zentrale Motor (4) ein Gehäuse zum Schutz vor Feuchtigkeit umfassen.

## Revendications

1. Dispositif automatisé de comptage de pupes à haute résolution qui comprend :
- une plateforme (1),
- un ou plusieurs porte-tubes (2) agencés sur la plateforme (1),
- un ou plusieurs tubes à échantillon (3), insérés dans chaque porte-tube (2) et destinés à contenir des pupes,
- une caméra (6) pour l'enregistrement de vidéo et d'image, dirigée vers la plateforme (1), connectée à un dispositif externe qui quantifie le temps et le nombre de pupes contenues dans chaque tube à échantillon (3),
- un moteur central (4) relié à la plateforme,
**caractérisé en ce que** le moteur central (4) met en rotation la plateforme (1) jusqu'à ce qu'un tube à échantillon (3) d'intérêt soit positionné devant la caméra (6), **en ce que** le dispositif comprend en outre :
- un moteur externe (5) positionné à côté de la plateforme (1), qui met en rotation le tube à échantillon (3) d'intérêt à 360° sur lui-même devant la caméra (6).

2. Dispositif selon la revendication 1, dans lequel la plateforme (1) est circulaire et les porte-tubes (2) sont agencés en cercle.

3. Dispositif selon la revendication 1, dans lequel le moteur central (4) et/ou le moteur externe (5) sont des moteurs pas à pas.

4. Dispositif selon la revendication 1, dans lequel il comprend en outre des roulements, positionnés entre les porte-tubes (2) et la plateforme (1).

5. Dispositif selon la revendication 1, dans lequel il comprend en outre un module d'éclairage, qui illumine les tubes à échantillon (3).

6. Dispositif selon la revendication 5, dans lequel le module d'éclairage comprend une lumière infrarouge et une lumière blanche.

7. Dispositif selon la revendication 5 ou 6, dans lequel le module d'éclairage comprend un boîtier pour une protection contre l'humidité.

8. Dispositif selon la revendication 1, dans lequel le moteur externe (5) et le moteur central (4) comprennent un boîtier pour une protection contre l'humidité.
